# EUROPEAN PATENT SPECIFICATION

(11) **EP 0 715 563 B1**
(45) Date of publication and mention of the grant of the patent: **29.10.1997**
(21) Application number: 94926146.5
(22) Date of filing: 04.08.1994
(51) Int. Cl.: B26D 3/08, B26D 7/18, B26F 1/10

(54) **DEVICE TO PUNCH PLASTER TAPES**
LOCHVORRICHTUNG FÜR PFLASTER
DISPOSITIF POUR PERFORER DES BANDES DE PLATRE

(30) Priority: 25.08.1993 IT TO930627
(43) Date of publication of application: 12.06.1996
(73) Proprietor: Plastod S.P.A., 40012 Lippo di Calderara di Reno (IT); Dotta, Angelo, I-40125 Bologna (IT)
(72) Inventor: DOTTA, Angelo, I-40125 Bologna (IT)
(74) Representative: Robba, Eugenio
(86) International application number: EP9402579
(87) International publication number: WO9505927

(56) References cited:
- EP-A- 0 065 179
- DE-A- 2 461 789
- DE-A- 2 834 246
- FR-E- 79 264
- GB-A- 909 634
- GB-A- 1 245 444
- US-A- 1 286 377
- US-A- 1 965 893
- US-A- 2 264 134
- US-A- 3 073 304
- US-A- 3 566 726
- US-A- 4 370 942
- US-A- 4 441 395

## Description

The present invention deals with a device to punch plaster tapes.

It is well known that the plaster surface must be generally equipped with perforations to allow the skin to perspire.

GB-A-90̸9 634 discloses an apparatus for piercing a strip of film material comprising a first member having a layer of kneadable cold-flowing composition carried on a surface therof, means for positioning said material at said surface of said first member, a second member having a plurality of extending fingers, means for relatively moving said first and second members for penetration of said layer and the film in contact therewith by said fingers and means for kneading the surface of said layer to close the openings formed by said fingers.

It is common to produce plasters starting from tape reels including a first layer of support material, equipped with an adhesive side, and a second layer of protective material, for example siliconated paper, that can be easily separated from the first layer, commonly known as "liner".

The above-said layers are coupled either through the adhesive present on the first layer, or through other means, leaving, in this latter case, the adhesive surface of the first layer free.

After punching, the two layers are separated and subjected to other working steps.

In order to obtain the desired punching, two methods can be used, that can be respectively defined as hot method and cold method.

The term hot method deals with the tape punching technique that provides a slight penetration of heated needles that melt the adhesive support, if thermoplastic, and preserve the "liner" substantially integral, thus allowing it to be used again.

However, through this method, scarcely visible holes are obtained, that can be unequal, irregular and wrinkled due to the uneven thermoplastic melting when the distance from the hot needle point increases.

The term cold method deals with the tape punching technique that provides the use of cutting means that engrave the tape, generating circular holes and removing chips of material including both plaster and "liner", that therefore, being riddled as a result, is usually rejected.

Furthermore, this method is usually carried out through cutting means with respect to a hard contrast surface that therefore wears them and forces the cutting surfaces to be frequently replaced and sharpened.

Therefore, these surfaces are generally obtained through removable bodies to make their replacement easier.

These embodiments however are complex and usually allow obtaining punching densities that are not greater than 10̸ holes per cm².

Object of the present invention is providing a device to generate holes in the plaster surface that allows cold operations, without appreciably engraving the "liner", keeping it reusable, and that allows obtaining at the same time holes that are even and free from flaws.

Another object of the present invention is obtaining a device that allows strongly increasing the cutting surface life.

These and other objects are obtained through the device to punch plaster tapes, wherein an adhesive support layer and a layer of protective material are present, including an hollow rotating tubular body having on the surface thereof a plurality of drilled socket punches, communicating with the rotating body recess, radially arranged at the same distance from the rotation axis of the rotating body, equipped with a cutting edge at the hole mouth and contrast and drive means cooperating with said rotating body, the plaster tape being provided passing between said rotating body and said contrast means synchronously progressing with the rotating body, the device being characterized in that:
said socket punches form a single body with the rotating body or, alternatively, with removable sectors thereof;
the cutting edges of socket punches and contrast means are kept spaced, said distance being not greater than the thickness of the layer of protective material;
the hole inside the socket punches shows, starting from the cutting edges, a section whose diameter and length are respectively not greater and not lower than the cutting edge diameter;
small stacks of chips are present inside the socket punch holes to cooperate, when removing chips of protective material, with the cutting edges and the hole inside the socket punches.

A second embodiment of the device provides the stiff connection of the socket punches into suitable seats provided in the rotating body, or alternatively, in suitable seats provided in removable sectors of said rotating body.

A preferred, but not exclusive, embodiment of the invention will now be described with reference to the enclosed drawings in which:
Fig. 1 is a side view, partially in section, of the device;
Fig. 2 is a section of a part in the device in Fig. 1;
Fig. 3 is an axial view, partially in section, of the device in Fig. 1;
Fig. 4 is a partial view of a part in a second embodiment of the device;
Fig. 5 is a partial view of a part in a third embodiment of the device.

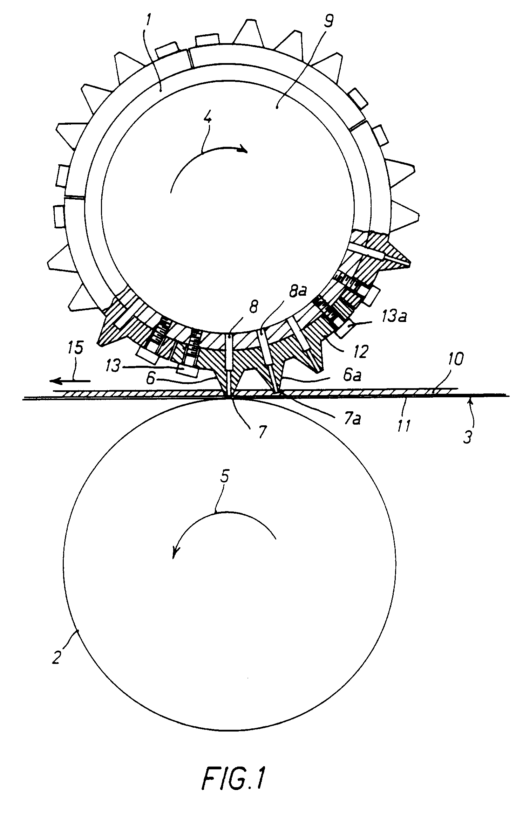

With reference to Figure 1, the device of the present invention includes a punching cylinder 1, equipped with an internal recess 9, on which there are assembled, through screws 13, 13a, the removable members 12 wherein, in a single body, the radial cone-shaped socket punches 6, 6a equipped with the cutting edges 7, 7a are formed.

Said cutting edges 7, 7a are kept spaced with respect to the surface of the smooth contrast cylinder 2 in order to allow the socket punches 6, 6a to penetrate into the plaster tape 3 till they reach the "liner" layer 11, without however completely punching it, through the adhesive support layer 10̸.

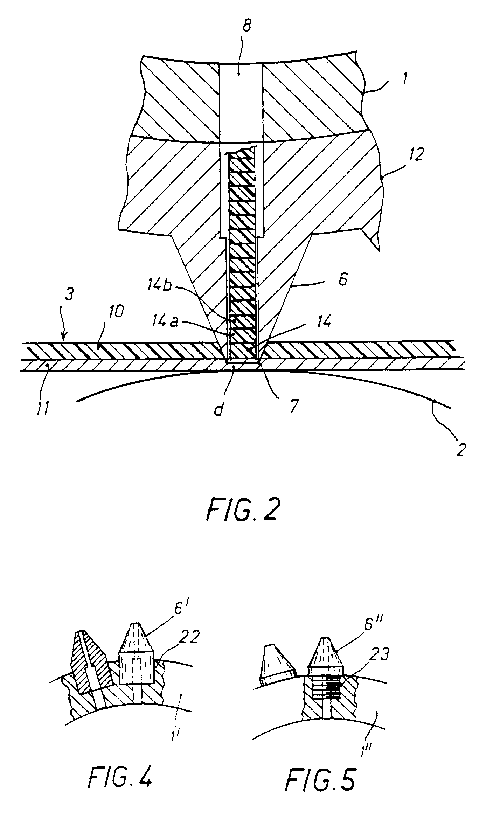

For this purpose, as detailed in figure 2, the distance "d" between the cutting edges 7 and the smooth surface of the contrast cylinder 2 is kept not lower than 50̸% of the thickness of the "liner" layer 11.

During rotation of the cylinder 1, therefore, punching of the first layer 10̸ and the sole engraving of the second layer 11 are carried out followed by the removal of a chip of material coming from the first layer 10̸ only.

The chips 14, 14a, 14b thus formed adhere one on top of the other due to the adhesive, both if it is facing the layer 11 and if it is on the free surface of the layer 10̸ and is therefore facing the socket punch 6.

The small stacks of chips therefore cooperate with the surface of the hole 8 , inside the socket punch, in punching the tape, making removal of the following chips easier during rotation of the cylinders 1 and 2.

Said rotation occurs in the direction shown by arrows 4 and 5, and favours the tape translation in the direction shown by arrow 15.

The hole 8 of the socket punch 6 shows a first section, next to the cutting edges, whose diameter and length are respectively not greater and not lower than the diameter of the cutting edge and a second section, facing the cylinder recess, whose diameter is greater than the first section.

In other embodiments that provide the use of highly resistant steels, the hole inside the socket punches shows a single section directly communicating with the internal recess 9 of the punching cylinder 1.

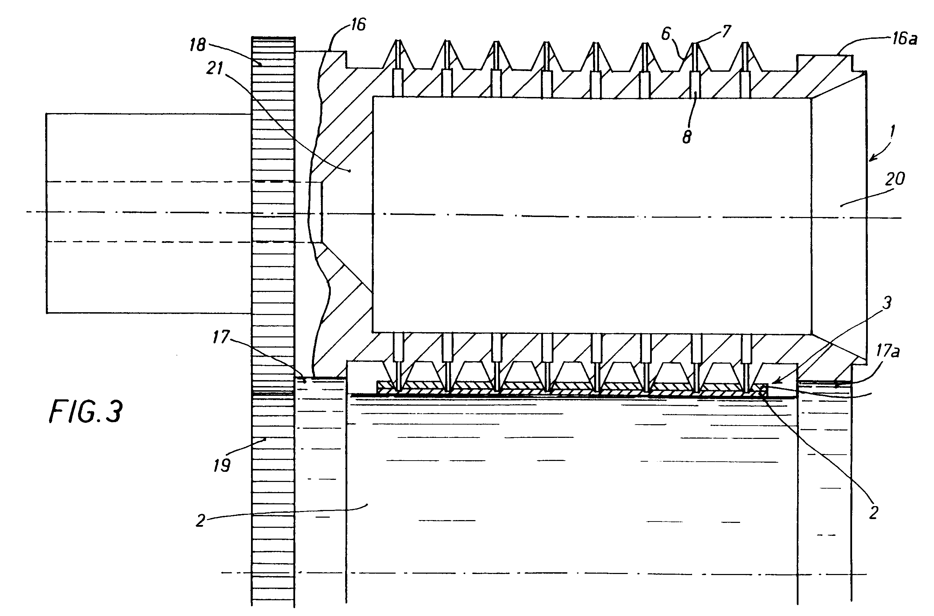

With reference to Figure 3, the punching cylinder 1 is further equipped with "shoulders" 16, 16a opposed to other contrast "shoulders" 17, 17a provided on the cylinder 2, suitable to keep cylinders 1 and 2 spaced, that are counter-rotating by means of the mating of gears 18 and 19.

The cylinder 1 can further by equipped with a side opening 20̸ and with a cone-shaped duct 21 which communicates with a source of compressed air, not shown, adapted to make chips removal easier.

The described device is suitable to operate with the maximum accuracy and to drill very small holes (from 0̸.5 mm to 1.0̸ mm in diameter) and closely spaced (20̸ holes per cm²).

Furthermore, the chips are completely removed and are not scattered on the tape being worked making it unusable.

In order to obtain these results, in addition to a very accurate adjustment of the distance between the cylinders, that can be obtained, as described, by means of the "shoulders", it is further necessary that the short elastic stacks of layered chips be present inside the socket punch holes.

Said short stacks, upon start-up of the device, can be preformed by punching a single-layer tape, with adhesive support only, and by using as contrast means a cylinder made of wood or other material against which the cutting edges of the socket punches can operate without being damaged.

With reference to Figure 4, the socket punch 6' is stiffly connected, for example by pressure and with the help of welding means, in circular seats 22 provided in the body of cylinder 1'.

Alternatively, the socket punches can be stiffly connected, as described in the previous paragraph, into suitable seats formed in the removable members 12.

With reference to Figure 5, the socket punch 6'' is equipped with external thread adapted to allow it to be screwed into threaded seats 23 provided in the body of cylinder 1''.

Alternatively, the socket punches can be screwed, as described in the previous paragraph, into suitable threaded seats formed in the removable members 12.

## Claims

1. Device to punch plaster tapes, wherein an adhesive support layer (10) and a layer (11) of protective material are present, including an hollow rotating tubular body (1) having on the external surface thereof a plurality of drilled socket punches (6, 6a), communicating with the rotating body (1) recess, equipped with a cutting edge (7, 7a) at the external hole mouth, radially arranged at the same distance from the rotation axis of the rotating body (1), and contrast and drive means (2) cooperating with said rotating body (1), the plaster tape (3) being provided passing between said rotating body (1) and said contrast and drive means (2) synchronously progressing with the rotating body (1), characterized in that:
said socket punches (6, 6a) form a single body with the rotating body (1) or, alternatively, with removable sectors thereof;
the cutting edges (7, 7a) of socket punches (6, 6a) and contrast and drive means (2) are kept spaced, said distance being not greater than the thickness of the layer of protective material;
the hole (8) inside the socket punches (6, 6a) shows, starting from the cutting edges (7, 7a), a section whose diameter and length are respectively not greater and not lower than the cutting edge diameter; so that small stacks of chips (14, 14a, 14b) which are present inside the socket punch holes (8) cooperate, when removing chips of protective material, with the cutting edges (7, 7a) and the hole inside the socket punches (6, 6a).

2. Device to punch plaster tapes, wherein an adhesive support layer (10) and a layer (11) of protective material are present, including an hollow rotating tubular body (1) having on the external surface thereof a plurality of drilled socket punches (6, 6a), communicating with the rotating body (1) recess, equipped with a cutting edge (7, 7a) at the external hole mouth, radially arranged at the same distance from the rotation axis of the rotating body (1), and contrast and drive means (2) cooperating with said rotating body (1), the plaster tape (3) being provided passing between said rotating body (1) and said contrast and drive means (2) synchronously progressing with the rotating body (1), characterized in that:
said socket punches (6, 6a) are stiffly connected into suitable seats (22) provided in the rotating body (1), or alternatively into suitable seats (22) provided in removable sectors (12) of said rotating body; (1),
the cutting edges (7, 7a) of socket punches (6, 6a) and contrast and drive means (2) are kept spaced, said distance being not greater than the thickness of the layer of protective material;
the hole (8) inside the socket punches (6, 6a) shows, starting from the cutting edges (7, 7a), a section whose diameter and length are respectively not greater and not lower than the cutting edge diameter; so that small stacks of chips (14, 14a, 14b) which are present inside the socket punch holes (8) cooperate, when removing chips of protective material, with the cutting edges and the hole inside the socket punches (6, 6a).

3. Device as claimed in claim 2, characterized in that said socket punches (6, 6a) are stiffly connected into said seats (22) by pressure and with the help of welding means.

4. Device as claimed in claim 2, characterized in that said seats (22) provided in the rotating body (1) or alternatively in removable sectors (12) thereof, show a threaded surface and in that said socket punches (6, 6a) are also externally threaded.

5. Device as claimed in claim 1 or 2, characterized in that said tubular rotating body (1) is realized by means of a hollow cylinder (1) equipped with spacing reliefs or shoulders (16, 16a) adapted to avoid contact of the cutting edges (7, 7a) of the socket punches (6, 6a) with the contrast and drive means (2) when punching.

6. Device as claimed in claim 5, characterized in that the contrast and drive means (2) are realized by means of a cylinder (2) equipped with spacing shoulders (17, 17a) cooperating with said opposed spacing shoulders (16, 16a) provided in the hollow cylinder (1), said cylinders (1, 2) being counter-rotating with respect to parallel axes through peripheral gears (18,19).

7. Device as claimed in claim 5, characterized in that the distance between said cutting edges (7, 7a) and said contrast and drive means (2) is not lower than 50̸% of the thickness of the layer (11) of protective material.

8. Device as claimed in claim 5, characterized in that the chips (14, 14a, 14b) removed when punching the tape include a layer of adhesive support only.

9. Device as claimed in claim 5, characterized in that said hollow cylinder (1) shows an open side to eject the chips (14, 14a, 14b) deriving from punching and a side (20) in which a hole is present that widens inside the cylinder (1) in a cone-shaped duct (21), said hole communicating with a source of compressed air to eject the chips.

10. Device as claimed in claim 1 or 2, characterized in that the arrangement of said socket punches (6, 6a) on said tubular body (1) is adapted to produce in said tape a plurality of holes grouped in cyclically repeating configurations.

11. Device as claimed in claim 1 or 2, characterized in that the hole (8) inside the socket punches (6, 6a) shows a section, facing the rotating body (1) recess (9), whose diameter is greater than the section facing the cutting edges.

## Patentansprüche

1. Einrichtung zum Lochen von Bändern für Heftpflaster mit einer Haftschichte (10) und einer Schutzauflage, bestehend aus einem hohlen, drehbar gelagerten, rohrförmigen Körper (1) mit einer äußeren Oberfläche, an welcher eine Vielzahl von längsdurchbohrten Stanzbuchsen (6, 6a) angeordnet sind, die mit dem Hohlraum des Körpers (1) verbunden sind und die eine Schneidkante (7, 7a) an der äußeren Bohrungsmündung aufweisen, welche alle im selben Ausmaß von der Drehachse des Körpers (1) radial distanziert sind und Widerlager- und Antriebsmittel (2) mit dem Körper (1) zusammenwirken und das Band zwischen dem sich drehenden Körper (1) und den Widerlager- und Antriebsmitteln (2) durchgeführt ist und letztere synchron mit dem drehbar gelagerten Körper (1) sich bewegen, dadurch gekennzeichnet,
- daß die Stanzbuchsen (6, 6a) einstückig mit dem drehbar gelagerten Körper (1) ausgebildet sind oder aber mit von diesem abnehmbaren Sektoren;
- daß die Schneidkanten (7, 7a) der Stanzbuchsen (6, 6a) und die Widerlager- und Antriebsmittel (2) voneinander distanziert sind, wobei der Abstand nicht größer ist als die Stärke der Schutzauflage;
- daß die Bohrung (8) im Inneren der Stanzbuchsen (6, 6a), ausgehend von den Schneidkanten (7, 7a), einen Abschnitt aufweist, dessen Durchmesser bzw. Länge nicht größer und nicht kleiner ist als der Durchmesser der Schneidkante, so daß kleine Stapel von Stanzausschnitten (14, 14a, 14b) in den Bohrungen (8) der Stanzbuchsen vorhanden sind, die, wenn Stanzausschnitte von der Schutzauflage abgenommen werden, mit den Schneidkanten (7, 7a) und der Bohrung in den Stanzbuchsen (6, 6a) zusammenwirken.

2. Einrichtung zum Lochen von Bändern für Heftpflaster mit einer Haftschichte (10) und einer Schutzauflage, bestehend aus einem hohlen, drehbar gelagerten, rohrförmigen Körper (1) mit einer äußeren Oberfläche, an welcher eine Vielzahl von längsdurchbohrten Stanzbuchsen (6, 6a) angeordnet sind, die mit dem Hohlraum des Körpers (1) verbunden sind und die eine Schneidkante (7, 7a) an der äußeren Bohrungsmündung aufweisen, welche alle im selben Ausmaß von der Drehachse des Körpers (1) radial distanziert sind und Widerlager- und Antriebsmittel (2) mit dem Körper (1) zusammenwirken und das Band zwischen dem sich drehenden Körper (1) und den Widerlager- und Antriebsmitteln (2) durchgeführt ist und letztere synchron mit dem drehbar gelagerten Körper (1) sich bewegen, dadurch gekennzeichnet,
- daß die Stanzbuchsen (6, 6a) in korrespondierenden Sitzen (22) festgelegt sind, die im drehbar gelagerten Körper (1) ausgebildet sind oder aber in korrespondierenden Sitzen (22), die in abnehmbaren Sektoren (12) des drehbar gelagerten Körpers (1) angeordnet sind;
- daß die Schneidkanten (7, 7a) der Stanzbuchsen (6, 6a) und die Widerlager- und Antriebsmittel (2) voneinander distanziert sind, wobei der Abstand nicht größer ist als die Stärke der Schutzauflage;
- daß die Bohrung (8) innerhalb der Stanzbuchsen (6, 6a), ausgehend von den Schneidkanten (7, 7a), einen Abschnitt aufweist, dessen Durchmesser bzw. Länge nicht größer und nicht kleiner ist als der Durchmesser der Schneidkante, so daß kleine Stapel von Stanzausschnitten (14, 14a, 14b) in den Bohrungen (8) der Stanzbuchsen vorhanden sind, die, wenn Stanzausschnitte von der Schutzauflage entfernt werden, mit den Schneidkanten und der Bohrung in den Stanzbuchsen (6, 6a) zusammenwirken.

3. Einrichtung nach Anspruch 2, dadurch gekennzeichnet, daß die Stanzbuchsen (6, 6a) in ihren Sitzen (22) durch eine Preßpassung und mit Hilfe von Schweißmitteln festgelegt sind.

4. Einrichtung nach Anspruch 2, dadurch gekennzeichnet, daß die Sitze (22), die im drehbar gelagerten Körper (1) oder aber in abnehmbaren Sektoren (12) desselben vorgesehen sind, ein Gewinde aufweisen, in welche die Stanzbuchsen (6, 6a) mit einem Außengewinde eingeschraubt sind.

5. Einrichtung nach Anspruch 1 oder 2, dadurch gekennzeichnet, daß der drehbar gelagerte, rohrförmige Körper (1) durch einen Hohlzylinder gebildet ist, der mit Distanzierungsmitteln oder Schultern (16, 16a) ausgebildet ist, die verhindern, daß die Schneidkanten (7, 7a) der Stanzbuchsen (6, 6a) beim Lochen die Widerlager- und Antriebsmittel (2) berühren.

6. Einrichtung nach Anspruch 5, dadurch gekennzeichnet, daß die Widerlager- und Antriebsmittel (2) durch einen Zylinder (2) gebildet sind, der mit Distanzierungsschultern (17, 17a) ausgestattet ist, die mit den gegenüberliegenden Distanzierungsschultern (16, 16a) zusammenwirken, die am Hohlzylinder (1) angeordnet sind, und die Zylinder (1, 2) um parallel zueinander liegende Achsen mittels außenliegenden Getrieben (18, 19) gegeneinander rotieren.

7. Einrichtung nach Anspruch 5, dadurch gekennzeichnet, daß der Abstand zwischen den Schneidkanten (7, 7a) und den Widerlager- und Antriebsmitteln (2) nicht kleiner ist als 50 % der Stärke der Schutzauflage (11).

8. Einrichtung nach Anspruch 5, dadurch gekennzeichnet, daß die Stanzausschnitte (14, 14a, 14b), die beim Stanzen vom Band abgenommen sind, ausschließlich eine Lage aus der Haftschichte aufweisen.

9. Einrichtung nach Anspruch 5, dadurch gekennzeichnet, daß der Hohlzylinder (1) eine offene Seite besitzt zum Auswurf der Stanzausschnitte (14, 14a, 14b), die von der Lochung stammen, und eine Seite (20) mit einer Höhlung, die innerhalb des Zylinders (1) in einen konischen Abschnitt (21) übergeht, wobei diese Höhlung mit einer Druckluftquelle zum Ausblasen der Stanzausschnitte verbunden ist.

10. Einrichtung nach Anspruch 1 oder 2, dadurch gekennzeichnet, daß die Anordnung der Stanzbuchsen (6, 6a) am rohrförmigen Körper (1) geeignet ist, um im Band eine Vielzahl von Ausstanzungen herzustellen, die in zyklischen Rapporten angeordnet sind.

11. Einrichtung nach Anspruch 1 oder 2, dadurch gekennzeichnet, daß die Bohrungen (8) innerhalb der Stanzbuchsen (6, 6a) an der dem Hohlraum (9) des drehbar gelagerten Körpers (1) zugewandten Seite einen Abschnitt aufweisen, dessen Durchmesser größer ist als der Durchmesser jenes Abschnittes, der den Schneidkanten zugewandt ist.

## Revendications

1. Dispositif pour perforer des bandes de plâtre, dans lesquelles une couche de support adhésif (10) et une couche (11) de matériau de protection sont présentes , comprenant un corps tubulaire rotatif creux (1) présentant sur sa surface externe une pluralité d'outils de perforation percés d'un trou (6,6a), communiquant avec le creux du corps rotatif (1) muni d'un bord de coupe (7,7a) au niveau de l'ouverture externe du trou , agencés radialement à la même distance de l'axe de rotation du corps rotatif (1), et des moyens d'entraînement et d'inversion (2) coopérant avec ledit corps rotatif (1), la bande de plâtre (3) étant prévue pour passer entre ledit corps rotatif (1) et lesdits moyens d'entraînement et d'inversion (2) avançant en synchronisation avec le corps rotatif (1), caractérisé en ce que :
lesdits outils de perforation à trous (6,6a) forment un corps unique avec le corps rotatif (1) ou, en variante, avec les secteurs mobiles de ce corps ;
les bords de coupe (7,7a) des outils de perforation à trous (6,6a) et les moyens d'entraînement et d'inversion (2) sont maintenus espacés , ladite distance n'étant pas plus grande que l'épaisseur de la couche du matériau de protection ;
le trou (8) formé à l'intérieur des outils de perforation à trous (6,6a) présente, à partir des bords de coupe (7,7a), une section dont le diamètre et la longueur ne sont pas, respectivement, supérieur et inférieure au diamètre du bord de coupe ; de sorte que
les petites piles de pastilles (14,14a, 14b) qui sont présentes à l'intérieur des trous des outils de perforation à trous (8) coopèrent , lors de l'enlèvement des pastilles de matériau de protection, avec les bords de coupe (7,7a) et le trou formé dans les outils de perforation à trous (6,6a).

2. Dispositif pour perforer des bandes de plâtre, dans lesquelles une couche de support adhésif (10) et une couche (11) de matériau de protection sont présentes, comprenant un corps tubulaire rotatif creux (1) comportant sur sa surface extérieure une pluralité d'outils de perforation percés d'un trou (6,6a) , communiquant avec l'évidement du corps rotatif (1) , prévus avec un bord de coupe (7,7a) au niveau de l'ouverture externe du trou , disposées radialement à la même distance de l'axe de rotation du corps rotatif (1) , et des moyens d'entraînement et d'inversion (2) coopérant avec ledit corps rotatif (1), la bande de plâtre (3) étant prévue pour passer entre ledit corps rotatif (1) et lesdits moyens d'entraînement et d'inversion (2) avançant en synchronisation avec le corps rotatif (1) caractérisé en ce que :
lesdits outils de perforation à trous (6,6a) sont connectés de façon rigide dans des embases appropriées (22) prévues dans le corps rotatif (1), ou en variante, dans des embases appropriées (22) prévues dans les secteurs amovibles dudit corps rotatif (1),
les bords de coupe (7,7a) des outils de perforation à trous (6,6a) et les moyens d'entraînement et d'inversion (2) sont maintenus espaces, ladite distance n'étant pas plus grande que l'épaisseur de la couche de matériau de protection;
le trou (8) formé à l'intérieur des outils de perforation à trous (6,6a) présente, à partir des bords de coupe (7,7a), une section dont le diamètre et la longueur ne sont pas , respectivement, supérieur et inférieure au diamètre du bord de coupe ; de sorte que
les petites piles de pastilles (14, 14a, 14b) qui sont présentes à l'intérieur des trous de perforation à trous (8) coopèrent , lorsqu'on enlève les pastilles de matériau de protection, avec les bords de coupe et le trou à l'intérieur des outils de perforation à trous (6,6a).

3. Dispositif selon la revendication 2, caractérisé en ce que lesdits outils de perforation à trous (6,6a) sont connectés de façon rigide dans lesdites embases (22) par pression et avec l'aide de moyens de soudure.

4. Dispositif selon la revendication 2, caractérisé en ce que lesdites embases (22) prévues dans le corps rotatif (1) ou, en variante, dans les secteurs (12) amovibles de celui-ci, présentent une surface taraudée et en ce que lesdits outils de perforation à trous (6,6a) sont également filetés extérieurement.

5. Dispositif selon la revendication 1 ou 2, caractérisé en ce que ledit corps tubulaire rotatif (1) est réalisé au moyen d'un cylindre creux (1) muni de parties saillantes séparées ou d'épaulements (16,16a) , adaptées pour éviter un contact des bords de coupe (7,7a) des outils de perforation à trous (6,6a) avec les moyens d'entraînement et d'inversion (2) lors de la perforation.

6. Dispositif selon la revendication 5, caractérisé en ce que les moyens d'entraînement et d'inversion (2) sont réalisés par l'intermédiaire d'un cylindre (2) équipé d'épaulements espacés (17,17a) coopérant avec lesdits épaulements d'espacement opposés (16,16a) prévus dans le cylindre creux (1) , lesdits cylindres (1,2) tournant dans le sens contraire par rapport aux axes parallèles au moyen d'engrenages périphériques (18,19).

7. Dispositif selon la revendication 5, caractérisé en ce que la distance comprise entre lesdits bords de coupe (7,7a) et lesdits moyens d'entraînement et d'inversion (2) n'est pas inférieure à 50 % de l'épaisseur de la couche (11) de matériau de protection.

8. Dispositif selon la revendication 5, caractérisé en ce que les pastilles (14, 14a, 14b) enlevées lors de la perforation de la bande comprennent seulement une couche de support adhésif.

9. Dispositif selon la revendication 5, caractérisé en ce que ledit cylindre creux (1) présente un côté ouvert pour éjecter les pastilles (14, 14a, 14b) provenant de la perforation et un côté (20) dans lequel se trouve un trou qui s'élargit à l'intérieur du cylindre (1) en un conduit de forme conique (21) , ledit trou communiquant avec une source d'air comprimé pour éjecter les pastilles.

10. Dispositif selon la revendication 1 ou 2, caractérisé en ce que l'agencement desdits outils de perforation à trous (6,6a) sur ledit corps tubulaire (1) est adapté pour produire dans ladite bande une pluralité de trous groupés selon des configurations répétées cycliquement.

11. Dispositif selon la revendication 1 ou 2, caractérisé en ce que le trou (8) formé à l'intérieur des outils de perforation à trous (6,6a) présente une section faisant face à l'évidement (9) du corps rotatif (1), dont le diamètre est supérieur, à la section faisant face aux bords de coupe .
